# EUROPEAN PATENT APPLICATION

(11) **EP 3 399 302 A1**
(43) Date of publication of application: **07.11.2018**
(21) Application number: 17753071.4
(22) Date of filing: 09.02.2017
(51) Int. Cl.: G01N 21/85, B07C 5/342, B65G 13/071, B65G 39/02, G01B 11/24

(54) **EGG SURFACE INSPECTION APPARATUS**

(30) Priority: 17.02.2016 JP 2016027563
(71) Applicant: Nabel Co., Ltd., Kyoto-shi Kyoto 601-8444 (JP)
(72) Inventor: KIYOTA, Akio, Kyoto-shi Kyoto 601-8444 (JP)
(74) Representative: Prüfer & Partner mbB Patentanwälte · Rechtsanwälte
(86) International application number: PCT/JP2017/004750
(87) International publication number: WO 2017/141813

(57) **Abstract**

An egg surface inspection apparatus (1) includes a conveying unit (2), a light projecting unit (3), an imaging unit (4) and a determination unit (5). The conveying unit (2) conveys an egg (E) while rotating the egg (E) with the egg's longitudinal axis serving as a rotation axis. The light projecting unit (3) projects linear light extending in a direction traversing a conveying direction (A) of the conveying unit (2). The imaging unit (4) recognizes the egg (E) irradiated with the linear light by the light projecting unit (3) as a two-dimensional image. The determination unit (5) determines a state of a surface of the egg (E) based on positional data of the linear light imaged by the imaging unit (4).

## Description

### TECHNICAL FIELD

The present invention relates to an egg surface inspection apparatus, and more particularly to an egg surface inspection apparatus which detects a flaw or deformation of an external shape of an eggshell.

### BACKGROUND ART

As one example of a method for detecting a flaw or deformation of an external shape of an eggshell, there has conventionally been known a method in which an egg is tapped with a tapper to make a sound which is in turn used to inspect whether the egg has a cracked, chipped or similarly damaged eggshell. However, it has been difficult to analyze relatively significantly cracked eggs by the sound made by tapping, and such cracked eggs have been overlooked in some cases.

Accordingly, as another method, for example, in Patent Document 1, a method using an image obtained by capturing an image of an egg is also adopted. According to the method proposed in Patent Document 1, before detecting whether an eggshell has a cracked surface, image processing is performed for distinguishing the eggshell from a background excluding the eggshell. After the image processing, complicated image processing is performed through various steps.

### CITATION LIST

### PATENT DOCUMENT

PTD 1: Japanese Patent Laying-Open No. 11-287763

### SUMMARY OF INVENTION

### TECHNICAL PROBLEM

A crack detection apparatus using a captured image as conventional has required complicated processing. An object of the present invention is to provide an egg surface inspection apparatus capable of detecting a flaw or deformation of an external shape of an eggshell without requiring such complicated processing.

### SOLUTION TO PROBLEM

In order to achieve the above object, the present invention takes the following approach: That is, an egg surface inspection apparatus according to the present invention is a surface inspection apparatus configured to inspect a surface of an egg while conveying the egg while rotating the egg, and comprises a conveying unit, a light projecting unit, an imaging means, and a determination unit. The conveying unit conveys the egg in a direction traversing the longitudinal axis of the egg while rotating the egg with the egg's longitudinal axis serving as a rotation axis. The light projecting unit irradiates the egg with a linear light extending in a direction traversing a conveying direction in which the conveying unit conveys the egg. The imaging unit images the egg irradiated with the linear light by the light projecting unit. The determination unit obtains positional data of the linear light imaged by the imaging unit and determines a state of a surface of the egg based on the positional data.

In the egg surface inspection apparatus according to the present invention, preferably, in the conveying unit, the egg is rotated in a direction to roll in the conveying direction.

In the egg surface inspection apparatus according to the present invention, more preferably, the conveying unit has a pair of spool-shaped rollers spaced from and adjacent to each other in the conveying direction, and the egg is held by the pair of spool-shaped rollers.

In the egg surface inspection apparatus according to the present invention, still preferably, in the conveying unit, the spool-shaped rollers are each supported rotatably with a roller shaft serving as a rotation axis, and as the spool-shaped rollers each rotate in a direction to roll toward a side opposite to the conveying direction, the egg held by the pair of spool-shaped rollers rotates in a direction to roll in the conveying direction.

### ADVANTAGEOUS EFFECTS OF INVENTION

The present egg surface inspection apparatus can detect a comparatively large flaw or deformation of an external shape of an eggshell of a relatively significantly cracked egg or the like.

### BRIEF DESCRIPTION OF DRAWINGS

Fig. 1 is a perspective view showing a main part of an egg surface inspection apparatus according to an embodiment of the present invention.
Fig. 2 is a plan view showing a main part of the egg surface inspection apparatus according to the embodiment.
Fig. 3 is a front view showing a main part of the egg surface inspection apparatus according to the embodiment.
Fig. 4 is a schematic view showing a state in which an uncracked egg is observed by an imaging unit of the egg surface inspection apparatus according to the embodiment.
Fig. 5 is a schematic view showing a state in which a cracked egg is observed by the imaging unit of the egg surface inspection apparatus according to the embodiment.
Fig. 6 is a flowchart showing an example of a procedure of a process in a determination unit of the egg surface inspection apparatus according to the embodiment.
Fig. 7 is a plan view showing a main part of an egg surface inspection apparatus according to a modification of one embodiment of the present invention.
Fig. 8 is a front view showing a main part of the egg surface inspection apparatus according to the modification.

### DESCRIPTION OF EMBODIMENTS

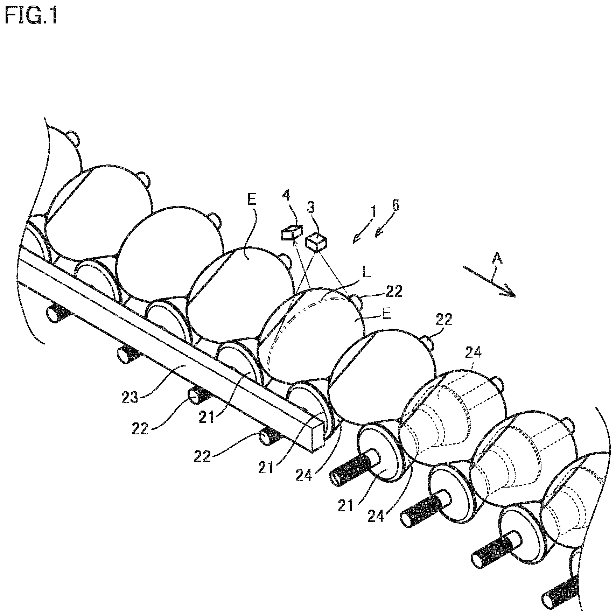

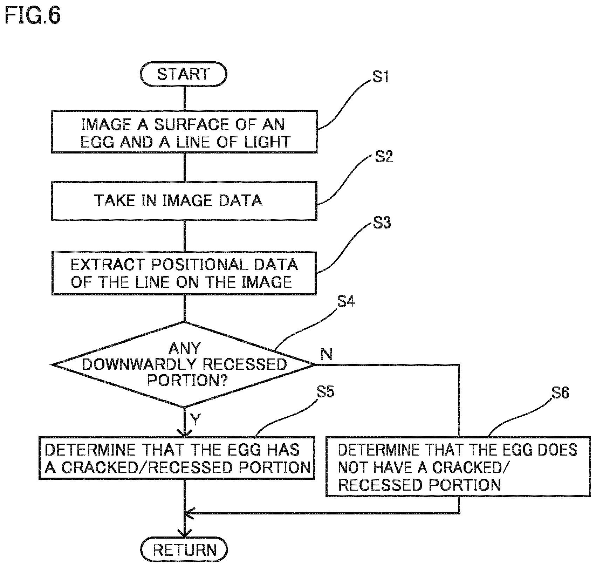

An egg surface inspection apparatus according to an embodiment of the present invention will be described with reference to Figs. 1 to 6.

An egg surface inspection apparatus 1 according to one embodiment is an inspection apparatus provided in an egg grading system (not shown) for detecting eggs having a relatively significantly cracked or recessed portion. As shown in Figs. 1 to 3, the apparatus inspects a surface of an egg E while conveying egg E with its longitudinal axis extending substantially horizontally and also substantially orthogonal to a direction A in which egg E is conveyed (or proceeds).

Egg surface inspection apparatus 1 includes a conveying unit 2, a light projecting unit 3, an imaging unit 4, and a determination unit 5. Conveying unit 2 conveys egg E while rotating egg E with the egg's longitudinal axis serving as a rotation axis. Light projecting unit 3 emits linear light extending in a direction substantially orthogonal to conveying direction A of conveying unit 2. Imaging unit 4 recognizes egg E irradiated with the linear light by light projecting unit 3 as a two-dimensional image. Determination unit 5 determines a state of a surface of egg E based on positional data of the linear light imaged by imaging unit 4. In the present specification, "conveying egg E while rotating egg E with the egg's longitudinal axis serving as a rotation axis" also includes conveying egg E while egg E is rotated without its longitudinal axis matching the actual rotation axis of egg E when egg E rotates.

As shown in Figs. 1 to 3, conveying unit 2 holds egg E by a pair of spool-shaped rollers 21 adjacent to each other in a frontward and rearward direction of conveyance and moves egg E downstream in conveying direction A. Conveying unit 2 is mainly composed of a plurality of spool-shaped rollers 21, a roller shaft 22, and a roller regulating unit 23. Eggs E are held by the plurality of spool-shaped roller 21. Roller shaft 22 is disposed at a predetermined interval. Spool-shaped roller 21 is attached to each roller shaft 22. Roller regulating unit 23 is provided at one end of roller shaft 22.

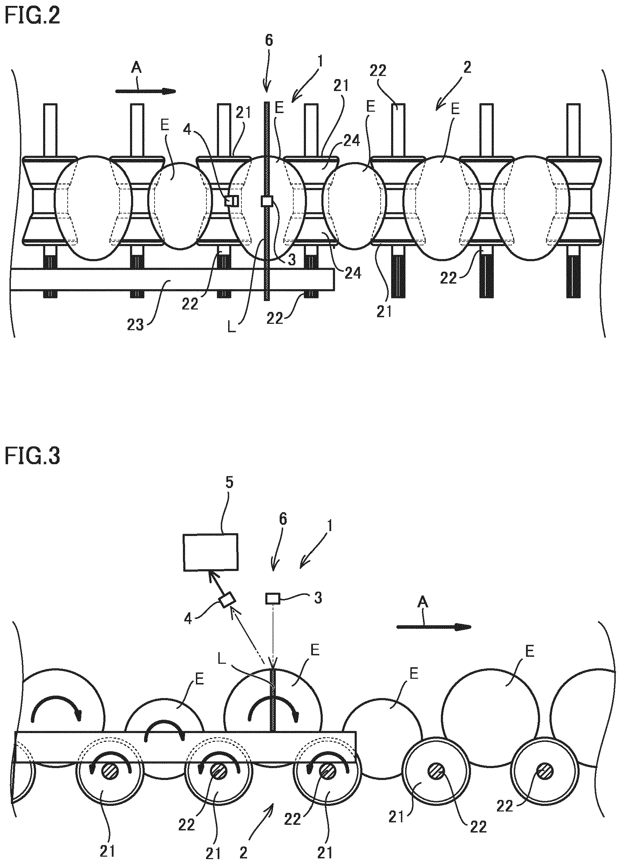

In particular, as shown in Fig. 3, in conveying unit 2, as viewed from a side where roller regulating unit 23 is disposed, each spool-shaped roller 21 rotates with roller shaft 22 serving as a rotation axis counterclockwise, as indicated in the figure by an arrow. As a result, egg E placed on spool-shaped roller 21 rotates clockwise as viewed opposite to the plane of the figure, as indicated in the figure by an arrow.

As shown in Figs. 1 to 3, spool-shaped roller 21 has a shape in which a vicinity of a center in a widthwise direction is recessed more than opposite ends in the widthwise direction. In the present embodiment, for example, six spool-shaped rollers 21 are fixed to roller shafts 22. In the figure, one of the six spool-shaped rollers 21 is shown representatively. Roller shaft 22 extends in a direction orthogonal to conveying direction A. Roller shaft 22 has opposite ends rotatably attached to a chain (not shown). A plurality of roller shafts 22 are disposed in parallel.

As shown in Figs. 1 to 3, roller regulating unit 23 is provided in conveying unit 2 at a predetermined position corresponding to a position where light projecting unit 3 and imaging unit 4 are disposed. Roller regulating unit 23 is disposed in contact with an upper portion of roller shaft 22. Frictional force is generated by roller regulating unit 23 brought into contact with roller shaft 22. Roller regulating unit 23, serving as a rotation imparting unit, applies its frictional force to roll roller shaft 22 and spool-shaped roller 21.

At the time, spool-shaped rollers 21 adjacent to each other in conveying direction A both rotate in the same direction, and egg E held by the adjacent spool-shaped rollers 21 is rotated in a direction opposite to that in which spool-shaped rollers 21 rotate. Egg E rolls in a direction to roll in conveying direction A. Note that spool-shaped roller 21 has an inclined portion 24 having a diameter larger than that in a vicinity of the center of the roller in the axial direction, and when spool-shaped roller 21 rotates, egg E is positionally adjusted so that egg E has its longitudinal axis substantially parallel to roller shaft 22 and egg E has its thick portion positioned at the center of the convey path.

As schematically shown in Fig. 1 to Fig. 3, light projecting unit 3 emits light toward egg E held by two adjacent spool-shaped rollers 21 and rotated. Light projecting unit 3 is disposed above conveying unit 2 and fixed to a supporting member (not shown). Fig. 1 shows a single egg E irradiated with light. However, for example, when eggs disposed in a plurality of rows are conveyed, a single light source may irradiate the plurality of rows of eggs with light.

Light projecting unit 3 emits light spreading in a two-dimensional sector. In light projecting unit 3, for example, a point light source and a cylindrical lens or the like (not shown) are combined together. By light projecting unit 3, a single line L (a line of light) is formed on conveying unit 2 and a surface of egg E located on conveying unit 2. Line L extends in a widthwise direction traversing conveying direction A. Line L extending in a straight line is formed substantially parallel to roller shaft 22.

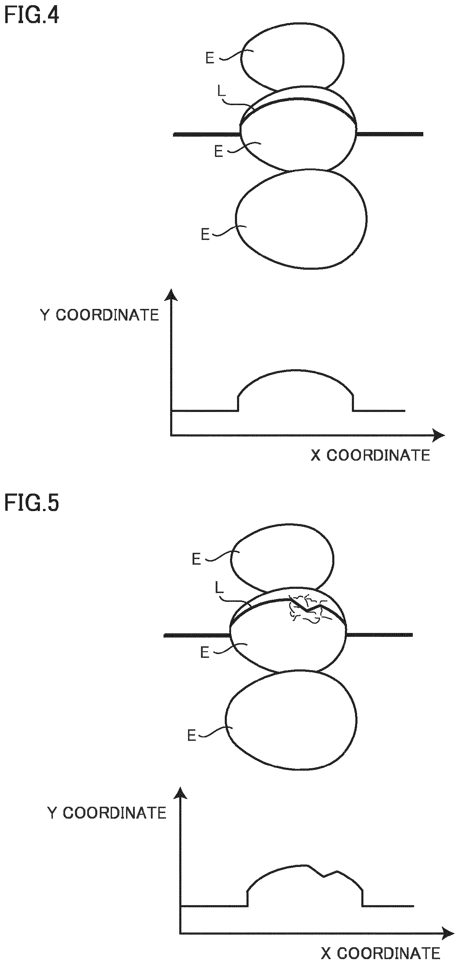

While line L of light emitted to a plane will be a straight line, line L of light emitted to egg E will be a curved line reflecting an external shape of egg E, as shown in Figs. 1, 4 and 5. As shown in Fig. 4, for an egg E which does not have a significantly cracked or recessed portion, a shape along a smooth shape of egg E appears.

On the other hand, as shown in Fig. 5, for an egg E which has a significantly cracked or recessed portion, line L of light is also bent as the portion is recessed. Figs. 4 and 5 each show a graph representing in the XY plane (or two dimensionally) positional data of line L of light irradiating egg E. The X coordinate corresponds to the widthwise direction traversing conveying direction A. The higher a level from a reference surface of egg E conveyed to an upper portion of egg E exposed to line L of light is, the larger the Y coordinate is.

As schematically shown in Figs. 1 to 3, imaging unit 4 is a camera provided at a position where a single line L of light projected on egg E can be imaged. Imaging unit 4 is disposed obliquely above the conveying device and upstream of light projecting unit 3 in conveying direction A, and fixed to a supporting member (not shown). An angle formed by a direction in which light projecting unit 3 emits light toward an egg and a direction toward imaging unit 4 from line L formed on a surface of the egg is set to a predetermined angle.

Based on the principle of triangulation, positional information of line L (a line of light) can be obtained from information of line L imaged by imaging unit 4. That is, a level of line L at each of different locations from the reference surface of egg E conveyed can be calculated. Information of a height of egg E calculated is recognized as information of an external shape (or surface shape) of egg E.

As schematically shown in Fig. 3, determination unit 5 determines a state of the surface based on the information of a shape of a line of light of an image captured. In determination unit 5, for example, a state of a surface of the eggshell of egg E imaged is determined depending on whether the positional data obtained from the line of light includes "positional data corresponding to a recessed or projecting portion which would not be calculated for an uncracked/unbroken, and accordingly normal egg." Determination unit 5 (an image processing means) may be anything that can determine a state of a surface of egg E based on a captured image, and is not limited to an embodiment described hereinafter.

Egg surface inspection apparatus 1 according to the present embodiment has each component controlled by a control device (not shown), which is a microcomputer system having a processor, a memory, an input interface, an output interface, and the like.

Hereinafter, an example of a method of inspecting a surface of an egg by using egg surface inspection apparatus 1 described above will be described.

Initially, egg E is placed on spool-shaped roller 21. Spool-shaped roller 21 with egg E placed thereon is moved in conveying direction A. When spool-shaped roller 21 moves, roller regulating unit 23 rotates spool-shaped roller 21. As spool-shaped roller 21 rotates, egg E rotates, and while rotating, egg E is conveyed to a detection position 6 where light projecting unit 3 and imaging unit 4 are disposed (see Figs. 1 to 3). At detection position 6, light projecting unit 3 continuously emits light in advance to form line L of light aimed at a surface of egg E conveyed to detection position 6.

Egg E conveyed to detection position 6 is irradiated with light and line L or the like accordingly projected on a surface of egg E is imaged by imaging unit 4 (step S1). Subsequently, data of the captured image is input to determination unit 5 (or the image processing means) (step S2). Determination unit 5 (the image processing means) extracts positional data of line L based on the data of the image (step S3).

Whether the extracted positional data corresponds to the positional data for a case where there is a portion recessed downward (or in a negative direction along the Y axis) (positional data F) is determined (step S4). When it is determined that the extracted positional data corresponds to positional data F, it is determined that egg E inspected has a cracked or recessed portion (step S5). In contrast, when it is determined that the extracted positional data does not correspond to positional data F, it is determined that egg E inspected does not have a cracked or recessed portion (step S6).

In step S4, although depending on the location, size and shape of a cracked or recessed portion in egg E, for example differentiating the positional data to calculate a slope of line L allows a determination to be more easily made.

In step S4, it may be determined whether the extracted positional data corresponds to the positional data for a case where there is a portion projecting upward (or in a positive direction along the Y axis) (positional data G). In that case, it can be determined whether egg E has a surface with a foreign matter adhering thereto.

A series of steps from imaging to making a determination, or an inspection, is repeated for the entire circumference of egg E by projecting light to different surfaces of egg E successively while rotating egg E. If it is determined while repeatedly performing the process for the entire circumference of egg E that "the egg has a cracked or recessed surface," a subsequent process for that egg E may be cancelled.

Thus egg surface inspection apparatus 1 according to the embodiment operates to inspect a surface of egg E while conveying egg E. Egg surface inspection apparatus 1 includes conveying unit 2, light projecting unit 3, imaging unit 4, and determination unit 5. Conveying unit 2 conveys egg E while rotating egg E with the egg's longitudinal axis serving as a rotation axis. Light projecting unit 3 emits linear light extending in a direction traversing conveying direction A of conveying unit 2. Imaging unit 4 recognizes egg E irradiated with the linear light by light projecting unit 3 as a two-dimensional image. Determination unit 5 determines a state of a surface of egg E based on positional data of the linear light imaged by imaging unit 4.

That is, egg surface inspection apparatus 1 described above employs a method referred to as a "light section method" to inspect a surface of an egg. The object to be inspected is irradiated with light in a line. The irradiated object reflects light and the reflection is obtained as positional data. Based on the obtained positional data of the reflected light, a surface of an egg is inspected.

When egg E has an eggshell having a relatively significantly cracked, chipped or recessed portion, egg E has an external shape which is significantly different from that of a normal egg. Accordingly, by using a linear light to obtain positional data regarding an external shape (or contour) of an egg, as described above, it can be determined whether the egg has a cracked, chipped, recessed or similarly damaged portion.

Egg surface inspection apparatus 1 described above processes a captured image of egg E to obtain positional data. In doing so, by determining a state of a surface of egg E based on positional data of linear light imaged, processing the image can be significantly easily done. This eliminates the necessity of performing a process to extract a feature, such as distinguishing an eggshell from background, as done in image-processing performed in a crack inspection apparatus as conventional.

An inspection of a surface of an egg in the light section method is also advantageous in that the inspection is less easily affected by a change of a gloss of an eggshell of an egg as moisture or the like adheres to the egg, or a difference in eggshell color between a white shell, a pink shell or a brown shell.

In particular, egg surface inspection apparatus 1 described above comprises conveying unit 2 that conveys egg E while rotating (or rolling) egg E. This allows positional data of a surface of egg E to be obtained for the entire circumference of the egg, and used to determine whether the egg has a cracked or recessed surface.

Furthermore, in conveying unit 2 of the egg surface inspection apparatus described above, egg E will rotate while being conveyed. At the time, egg E rotates (rolls) in a direction to roll in conveying direction A as spool-shaped roller 21 rotates. This allows egg surface inspection apparatus 1 comprising a single light projecting unit 3 and a single imaging unit 4 fixed thereto to inspect the entire circumferential surface of egg E.

Conventionally, a dirty egg inspection apparatus for inspecting dirt on an egg is also provided with a conveying unit which rotates the egg while conveying it. In this case, the egg is rotated in a direction to roll in a direction opposite to the conveying direction. In the dirty egg inspection apparatus, an egg is imaged by an imaging unit disposed at a plurality of locations from an upstream side toward a downstream side in the conveying direction.

Herein, it is assumed that whether an egg is dirty is inspected by a single light projecting unit and a single imaging unit both fixed to a virtual egg inspection apparatus. In that case, as viewed at the light projecting and imaging units fixed to the virtual inspection apparatus, a velocity of a surface portion of an egg irradiated with light and imaged will be a conveying velocity by the conveying unit (a velocity Vc) minus a velocity of a surface portion of the egg in a direction of a tangent to the surface portion as the egg rotates (a velocity Vd), (i.e., velocity Vc - velocity Vd).

Then, if there is no substantial difference between velocity Vc and velocity Vd, successively conveyed eggs will be imaged such that the eggs have their surfaces irradiated with light only at a limited circumferential position. For this reason, it is expected that it is impossible to reliably inspect that an egg is a dirty egg.

On the other hand, in conveying unit 2 of egg surface inspection apparatus 1, a direction of rotation of spool-shaped roller 21 and a direction of rotation of egg E are opposite to a direction of rotation corresponding thereto in the case of the conventional dirty egg inspection apparatus. More specifically, in conveying unit 2, roller shaft 22 travels in conveying direction A. Spool-shaped roller 21 rotates with roller shaft 22 as a rotation axis in a direction to roll in a direction opposite to conveying direction A. Egg E rotates in a direction to roll in a direction opposite to that in which spool-shaped roller 21 rotates, that is, in conveying direction A.

In that case, as viewed at light projecting and imaging units 3 and 4 fixed to egg surface inspection apparatus 1, a velocity of a surface portion of egg E irradiated with light will be a conveying velocity by conveying unit 2 (velocity Vc) plus a velocity of a surface portion of egg E in a direction of a tangent to the surface portion as egg E rotates (velocity Vd), (i.e., velocity Vc + velocity Vd).

This allows a single light projecting unit 3 to be used to irradiate successively conveyed eggs' entire circumferential surfaces with light and a single imaging unit 4 to be used to image the eggs' entire circumferential surfaces to obtain their positional data. This allows egg surface inspection apparatus 1 to be designed relatively compactly. In addition, this also allows egg surface inspection apparatus 1 to be manufactured at a reduced cost.

Note that even when an egg is rotated in a direction to roll in a direction opposite to the conveying direction, the egg can have its entire circumference inspected by egg surface inspection apparatus 1 provided with a light projecting unit and an imaging unit at each of a plurality of locations.

Further, conveying unit 2 of the above-described egg inspecting apparatus is spool-shaped roller 21 fixed to roller shaft 22. A pair of adjacent spool-shaped rollers 21 holds egg E. This allows egg E to be conveyed such that egg E has its longitudinal axis along a direction traversing the conveying direction (a conveying widthwise direction). In addition, a movement of egg E in the direction of the longitudinal axis of egg E (i.e., the conveying widthwise direction) will be restricted by inclined portion 24 of spool-shaped roller 21 located at one axial end thereof and inclined portion 24 of spool-shaped roller 21 located at the other axial end thereof. This can facilitate setting a conveying widthwise direction in imaging egg E by imaging unit 4.

In conveying unit 2, spool-shaped roller 21 can be rotated (rolled) by bringing roller shaft 22 into contact with roller regulating unit 23 disposed above roller shaft 22. Thus, spool-shaped roller 21 can be rotated in a desired direction by a simple structure by roller regulating unit 23, and egg E placed on spool-shaped roller 21 can also be rotated.

It should be noted that the present invention is not limited to the above-described embodiment.

The conveying unit is not limited to the above-described conveying unit 2 in so far as the conveying unit can convey an egg while rotating (rolling) the egg with its longitudinal axis in a predetermined direction (a lateral direction). Furthermore, while in the above-described embodiment conveying unit 2 is assumed to be conveying unit 2 arranged in six rows, the number of rows is not limited to 6 rows, and any number of rows, e.g., a single row, may be applied. Further, spool-shaped roller 21 may be replaced with a different, known roller or the like.

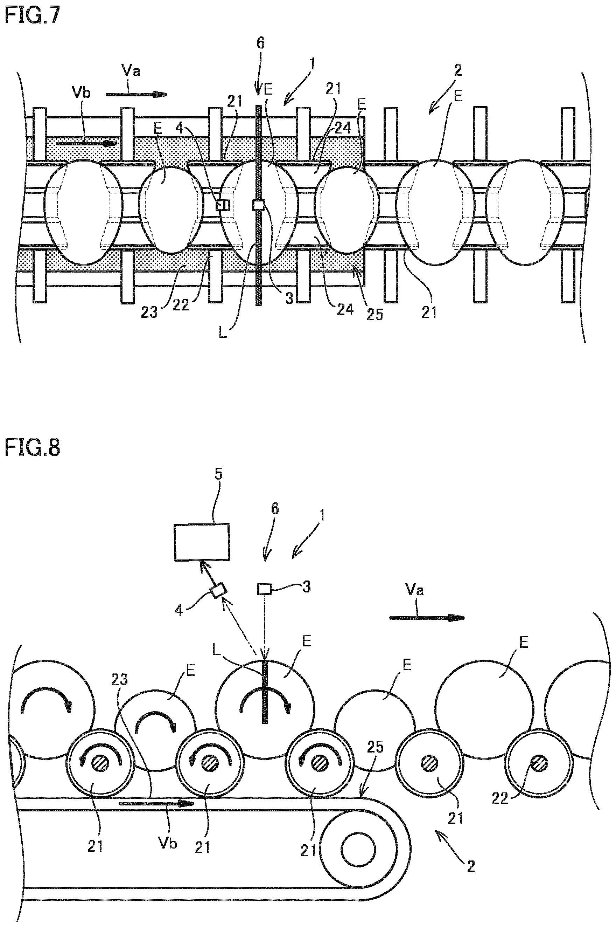

The roller regulating unit is not limited to that shown and may be changed variously in so far as it can rotate spool-shaped roller 21 to rotate (roll) an egg. For example, roller regulating unit 23 may be a belt conveyor 25 provided under spool-shaped roller 21, as shown in Figs. 7 and 8. Belt conveyor 25 has an upper surface brought into contact with spool-shaped roller 21 to cause frictional force to thus serve as a rotation imparting means for rotating spool-shaped roller 21. Note that in Figs. 7 and 8, any member identical to that of egg surface inspection apparatus 1 according to the above-described embodiment is identically denoted and will not be described repeatedly.

In conveying unit 2 according to this modification, spool-shaped rollers 21 each rotating about roller shaft 22 in a direction to roll in a direction opposite to the conveying direction will allow eggs E placed on spool-shaped rollers 21 to rotate in a direction to roll in the conveying direction.

Specifically, belt conveyor 25 is rotated in such a manner that belt conveyor 25 has its upper surface moved in the conveying direction. It suffices that a traveling velocity Vb of belt conveyor 25 is set to be larger than a traveling velocity Va of the conveying unit. In conveying unit 2, belt conveyor 25 will directly rotate the body of spool-shaped roller 21 having a diameter larger than the diameter of roller shaft 22. Accordingly, when an inspection is conducted by using light projecting and imaging units 3 and 4 fixed to the egg surface inspection apparatus, it is necessary to set traveling velocity Vb to be considerably faster than traveling velocity Va.

The light projecting unit is not limited to the above-described light projecting unit 3 in so far as the light emitted thereby and irradiating an egg can be detected by the imaging unit as a line (a line of light). As the light projecting unit, various types of light projecting units are applicable in so far as they are used for a so-called "light section method." For example, the above-described point light source may be substituted with a light projecting unit comprising a laser and a cylindrical lens or the like in combination. Furthermore, the light projecting unit is not limited to a light projecting unit which irradiates an egg with a single line of light, and a light projecting unit which emits light forming a plurality of lines is also applicable. Furthermore, a light projecting unit which emits light forming a streaky or similar pattern is applicable.

The determination unit (or a determination method) is not limited to the determination unit according to the above-described embodiment. For example, if it is difficult to distinguish which portion of a surface of an egg in data of a captured image of the egg is exposed to light and which portion thereof is not, the captured image may be binarized and a portion of a surface of the egg that is exposed to light may be extracted.

Furthermore, the method of detecting a downwardly recessed portion in positional data of a line (a line of light) is not limited to the above-described embodiment. For example, initially, an ideal external shape position estimated from each positional data in one image data, that can be assumed for an uncracked or unbroken external shape is calculated. Subsequently, this ideal external shape position is compared with a coordinate of actual positional data. If the actual positional data has a Y coordinate having a value smaller than that of the ideal external shape position, it can be determined that there is a downwardly recessed portion at the position of an X coordinate corresponding to that Y coordinate.

In contrast, if the actual positional data has a Y coordinate having a value (substantially) equal to that of the ideal external shape position, it can be determined that there is no recess or projection at the position of the X coordinate corresponding to that Y coordinate and that an uncracked or unbroken, and hence ideal external shape is had. Furthermore, if the actual positional data has a Y coordinate having a value larger than that of the ideal external shape position, it can be determined that there is an upwardly projecting portion at the position of the X coordinate corresponding to that Y coordinate.

Further, as another determination unit (or determination method), by utilizing the fact that an egg is imaged while being rotated, a downwardly recessed portion may be found from one image data obtained from the egg at one circumferential location and other image data obtained from the egg at another circumferential location. A downwardly recessed portion can be found by comparing positional data obtained from one image data with positional data obtained from other image data.

Further, average positional data of positional data obtained from image data of an uncracked or unbroken, and hence normal egg may be obtained in advance, and positional data obtained from one image data may be compared with the average positional data to find a downwardly recessed portion.

While in the above-described egg surface inspection apparatus, imaging unit 4 is disposed upstream of light projecting unit 3 in the conveying direction, imaging unit 4 may be disposed downstream of light projecting unit 3 in the conveying direction.

It should be understood that the embodiments disclosed herein have been described for the purpose of illustration only and in a non-restrictive manner in any respect. The present invention is defined by the terms of the claims, rather than the description above, and is intended to include any modifications within the meaning and scope equivalent to the terms of the claims.

### INDUSTRIAL APPLICABILITY

The present invention can be effectively applied to an egg surface inspection apparatus which detects a flaw or deformation of an external shape of an eggshell.

### REFERENCE SIGNS LIST

1: egg surface inspection apparatus; 2: conveying unit; 21: spool-shaped roller; 22: roller shaft; 3: light projecting unit; 4: imaging unit; 5: determination unit; E: egg.

## Claims

1. An egg surface inspection apparatus for inspecting a surface of an egg while conveying the egg while rotating the egg, comprising:
a conveying unit configured to convey the egg in a direction traversing a longitudinal axis of the egg while rotating the egg with the egg's longitudinal axis serving as a rotation axis;
a light projecting unit configured to irradiate the egg with a linear light extending in a direction traversing a conveying direction in which the conveying unit conveys the egg;
an imaging unit configured to capture an image of the egg irradiated by the light projecting unit with the linear light; and
a determination unit configured to obtain positional data of the linear light imaged by the imaging unit and determine a state of a surface of the egg based on the positional data.

2. The egg surface inspection apparatus according to claim 1, wherein in the conveying unit the egg is rotated in a direction to roll in the conveying direction.

3. The egg surface inspection apparatus according to claim 1 or 2, wherein
the conveying unit has a pair of spool-shaped rollers spaced from and adjacent to each other in the conveying direction, and
the egg is held by the pair of spool-shaped rollers.

4. The egg surface inspection apparatus according to claim 3, wherein in the conveying unit,
the spool-shaped rollers are each supported rotatably with a roller shaft serving as another rotation axis, and
as the spool-shaped rollers each rotate in a direction to roll toward a side opposite to the conveying direction, the egg held by the pair of spool-shaped rollers rotates in a direction to roll in the conveying direction.
